# EUROPEAN PATENT SPECIFICATION

(11) **EP 3 844 597 B1**
(45) Date of publication and mention of the grant of the patent: **21.10.2026**
(21) Application number: 18783093.0
(22) Date of filing: 29.08.2018
(51) Int. Cl.: G06F 3/01

(54) **MULTISENSORY HAPTIC SYSTEM AND METHOD**
MULTISENSORISCHES HAPTISCHES SYSTEM UND VERFAHREN
SYSTÈME ET PROCÉDÉ HAPTIQUES MULTISENSORIELS

(43) Date of publication of application: 07.07.2021
(73) Proprietor: ECOLE POLYTECHNIQUE FEDERALE DE LAUSANNE (EPFL), 1015 Lausanne (CH)
(72) Inventor: ROGNINI, Giulio, 1205 Geneve (CH); GALLO, Simone, 1273 Arzier (CH); BLANKE, Olaf, 1260 Nyon (CH)
(74) Representative: Grosfillier, Philippe
(86) International application number: PCT/IB2018/056590
(87) International publication number: WO 2020/044079

(56) References cited:
- EP-A1- 2 846 329
- EP-A2- 2 846 227
- EP-A2- 2 846 227
- US-A1- 2013 116 852
- US-A1- 2016 238 040
- W ANN ET AL: "Design and Quantization of FIR and IIR Filter for OFDM Signal", INTERNATIONAL JOURNAL FOR RESEARCH IN APPLIED SCIENCE & ENGINEERING TECHNOLOGY (IJRASET), vol. 5, no. IV, April 2017 (2017-04-01), pages 1251 - 1257, XP055577244, ISSN: 2321-9653

## Description

### Technical Field

The present invention is directed to the field of haptic devices and systems. In particular, the present invention concerns a multisensory system particularly suited for meditation and yoga training.

### Background

Contemplative practices have shown to be beneficial for many clinical conditions and to promote well-being. Scientific research and the Buddhist tradition highlight how these effects are based on specific neural, psychological, and phenomenological processes, in particular those related to the self. Current technological efforts focus more on the digital distribution of contemplative practice guidance (e.g. meditation apps), rather than on the support of the underlying processes that ground contemplative experiences to the self. A powerful approach to empirically investigate the self has been to target the brain mechanisms that process bodily signals (i.e. bodily self-consciousness/awareness). Recent developments in virtual reality and robotic technologies have allowed to investigate those mechanisms by providing subjects with ambiguous multisensory information about their own body. Similar paradigms have shown to restore embodiment and reduce neuropathic pain in different patient populations - including amputation and spinal cord injury - and to differentially affect expert meditators and healthy subjects (at both behavioural and neural level), suggesting a direct link between contemplative practices and bodily self-consciousness/awareness. Some examples of prior art includes EP2 846 227 A2 and EP2 846 329 A1 that disclose systems and methods for generating haptic effects by processing audio signals and converting them into control profiles.

The capability to focus on one's own bodily sensations is at the core of contemplative practices. This is shown by classical techniques such body scanning (i.e. progressively orienting one's own one attention towards different body parts) and breathing techniques (i.e. focus on the physical sensations linked to one's own breath) that uses bodily sensations as anchor points to increase focus and "clear up" the mind. Thus, guidance offered by expert meditators typically encourages practitioners to focus on tactile sensations occurring in their body, such as tingling, warmth, cold, pressure, etc. Interestingly, a particular emphasis is put on body parts that are in contact with the environment (thereby constantly receiving tactile feedback) such as the feet, in contact with the floor, or other body parts contacting the cushion or chair using during the practice. Yet, due to their lack of variability, the saliency of these sensations of contact fades away with time during the practice, whereas other tactile sensations in the body are not always present. While noisy environments (i.e. too much sensory stimulation) would distract the practice, too little and constant, not variable, exposure to sensory stimuli hinders the capability to focus in the present moment and increases boredom and mind wondering, especially in beginners.

The positive effects of meditation are due to the daily practice, yet acquiring this habit poses challenges. Dropout rate in non-patient population has been shown to reach 36% after only 3 months (Delmonte M.M., "Personality Characteristics and Regularity of Meditation," Psychological Reports Vol. 46, No. 3, pp. 703-712, 1980). To date, up to the inventors' knowledge, no technology is capable to support meditation by modulating bodily sensations during the practice with a multisensorial approach.

### Summary of the invention

According to some aspects of the present invention, as described hereinafter and in the appended claims, a goal is to overcome the drawbacks of the prior approaches as described.

Disclosed herein are systems and methods particularly suitable and adapted to support meditation practices based on a technology-mediated interaction with the bodily self. The used approach includes the personalized administration of complex tactile stimulations through a soft robotic interface in combination with auditory feedbacks to create immersive experiences.

According to some aspects of the disclosed invention, meditation practices and/or Yoga training are supported in two main ways: 1) by recreating the ideal sensory meditation scenario: a rich, engaging, but quite environment, that is, to re-create the immersive experience of meditating in a peaceful naturalistic environment such as a sea shore, and 2) by empowering meditation/Yoga expert's guidance, by connecting a (pre-recorded or live) guidance to the tactile stimulation actually perceived by the practitioner, thus favouring the practitioner to experience what the expert meditator encourages to feel.

Accordingly, one aspect of the present invention relates to a computer-implemented method and a computer readable medium having computer instruction code recorded thereon, the computer instruction code configured to perform a method of operating a haptic device when executed on a computer, the haptic device comprising a plurality of tactile displays configured to provide haptic stimuli to a user, the method comprising the steps of:
a) processing an audio signal derived from an audio file, thereby obtaining at least one profile of frequencies and amplitudes of the audio signal;
b) converting the frequencies and amplitudes profile into a haptic profile; and
c) operating the haptic device according to the haptic profile.

Another aspect of the present invention relates to a multisensory system comprising:
a) a haptic device comprising a plurality of tactile displays configured to provide haptic stimuli to a user;
b) a data processing apparatus operatively connected with the haptic device, the apparatus comprising a processor configured to perform the computer-implemented method as described herein; and
c) at least one audio device configured to reproduce an audio file.

Still another aspect of the present invention relates to the use of the multisensory system as described herein for Yoga training, meditation, massage or hypnosis' induction.

Still a further aspect of the present invention relates to method for performing Yoga training, meditation, massage or hypnosis' induction, the method comprising the steps of:
a) allowing a user to contact the multisensory system as described herein;
b) reproducing an audio file via the audio device of the multisensory system; and
c) operating the haptic device of the multisensory system
wherein the reproduced audio file is the same used for obtaining a haptic profile.

The above and other objects, features and advantages of the herein presented subject-matter will become more apparent from a study of the following description with reference to the attached figures showing some preferred aspects of the subject-matter.

### Brief Description of the Drawings

Figure 1 shows a schematic representation of an embodiment of the device according to an aspect of the invention;
Figure 2 presents a schematic sketch of two display types (one with a single display cell and another with multiple cells), in the inflated and resting states;
Figure 3 shows a schematic view of another embodiment of the system;
Figure 4 depicts one embodiment of the device adapted to be used under the feet of a user in e.g. Yoga training or meditation, according to an aspect of the present invention;
Figure 5 shows an illustration of the use of the multisensory system in the frame of Yoga training or meditation, according to an aspect of the present invention;
Figure 6 shows a schematic view of the various components of the multisensory system that empower meditation and guidance;
Figure 7 shows a similar embodiment with regards to Figure 6, in which the various components of the multisensory system are set to empower Yoga guidance. The only difference is that in the case of Yoga, the guidance, i.e. the different Yoga poses, can also be seen by the practitioners;
Figure 8 shows the various steps of the method according to another aspect of the present invention, in particular the audio signal processing and audio-to-tactile conversion;
Figure 9 shows the conversion of waves rise time to a spatio-temporal tactile stimulation pattern, according to yet another aspect of the present invention.

### Detailed Description of the Several Embodiments

The subject-matter herein described will be clarified in the following by means of the following description of those aspects which are depicted in the drawings. It is however to be understood that the subject matter described in this specification is not limited to the aspects described in the following and depicted in the drawings; to the contrary, the scope of the subject-matter herein described is defined by the claims. Moreover, it is to be understood that the specific conditions or parameters described and/or shown in the following are not limiting of the subject-matter herein described, and that the terminology used herein is for the purpose of describing particular aspects by way of example only and is not intended to be limiting.

Unless defined otherwise, technical and scientific terms used herein have the same meaning as commonly understood by one of ordinary skill in the art of the same technological field. Further, unless otherwise required by the context, singular terms shall include pluralities and plural terms shall include the singular. The methods and techniques of the present disclosure are generally performed according to conventional methods well known in the art and as described in various general and more specific references that are cited and discussed throughout the present specification unless otherwise indicated.

The present disclosure will be better understood with the help of the following definitions.

As used herein and in the appended claims, the singular forms "a", "an" and "the" include plural referents unless the context clearly dictates otherwise. Also, the use of "or" means "and/or" unless stated otherwise. Similarly, "comprise", "comprises", "comprising", "include", "includes" and "including" are interchangeable and not intended to be limiting. It is to be further understood that where descriptions of various embodiments use the term "comprising", those skilled in the art would understand that in some specific instances, an embodiment can be alternatively described using language "consisting essentially of" or "consisting of."

A "haptic device" is any device that exploits haptic technology. As used in the present disclosure, "haptic technology" or "haptics" is a feedback technology which recreates or stimulates the sense of touch by applying forces, pressures, temperatures, electrostimulations, vibrations and/or motions to the user. This mechanical stimulation can be used e.g. to assist in the creation of virtual objects in a computer simulation, to control such virtual objects, and to enhance the remote control of machines and devices (telerobotics). Haptic devices may incorporate sensors that measure forces, pressures or movements exerted by the user on the interface and vice versa.

As a research tool, haptic devices contribute to the understanding of how touch and its underlying brain functions work. A haptic device usually comprises a tactile display, a display device that presents information in tactile form (tactile feedbacks). The most common applications of the haptic technology include the provision of haptic feedbacks for controllers such as game controllers, joysticks, remote-controlled robotic tools, mobile devices such as mobile phones, virtual reality systems and so forth. Haptic interfaces for medical simulation may prove especially useful for e.g. training in minimally invasive procedures, as well as for performing remote surgery.

The term "multimodal" refers herein to the characteristic way by which a haptic device according to the present disclosure provides a user with a feedback. In particular, a multimodal feedback allows a user to experience multiple modes of interfacing with the haptic device. Multimodal interaction is the interaction with a virtual and/or a physical environment through natural modes of communication. This interaction enables a more free and natural communication, interfacing users with automated systems in both input and output. However, in the frame of the present disclosure, the term multimodal refers more specifically to the several modes by which the haptic device can provide tactile feedbacks to a user. The human sense of touch can be divided into two separate channels. Kinaesthetic perception refers to the sensations of positions, velocities, forces and constraints that arise from the muscles and tendons. Force-feedback devices appeal to the kinaesthetic senses by presenting computer-controlled forces to create the illusion of contact with a rigid surface. The cutaneous class of sensations arise through direct contact with the skin surface. Cutaneous stimulation can be further separated into the sensations of pressure, stretch, vibration, and temperature. Tactile devices generally appeal to the cutaneous senses by skin indentation, vibration, stretch and/or electrical stimulation. The device is construed and assembled in order to provide a tactile feedback involving one or more, possibly combined, among kinaesthetic or cutaneous sensations.

Multimodal devices can offer a flexible, efficient and usable way to allow users to interact through input modalities and to receive information by the device through output modalities. The multimodal device has to recognize the inputs from the different modalities, combining them according to temporal and contextual constraints in order to allow their interpretation, interpreting the fused inputs and returning to the user outputs arranged according to a consistent feedback.

A "closed-loop system", also known as a feedback control system, refers herein to a control system which uses the concept of an open loop system (in which the output has no influence or effect on the control action of the input signal) as its forward path but has one or more feedback loops (hence its name) or paths between its output and its input. The reference to "feedback" means that some portion of the output is returned back to the input to form part of the system's excitation or control. Closed-loop systems are usually designed to automatically achieve and maintain the desired output condition by comparing it with the actual condition. It does this by generating an "error" signal which is the difference between the output and the reference input. In other words, a closed-loop system is a fully automatic control system in which its control action is dependent on the output in some way.

As used herein, a "fluid" is a substance that continually deforms (flows) under an applied shear stress. Fluids are a subset of the phases of matter and include liquids, gases, plasmas and plastic solids. They display properties such as not resisting deformation, or resisting it only lightly and the ability to flow (also described as the ability to take on the shape of the container).

According to some of the embodiments, the fluid comprises or consists of a gas or preferably a liquid such as e.g. water, aqueous solutions, non-polar (e.g. oil) solutions and the like. An "aqueous solution" is a solution in which the solvent is substantially made of water. In the frame of the present disclosure, the term "aqueous" means pertaining to, related to, similar to, or dissolved in water. In the frame of the present disclosure, the fluid is also interchangeably referred to as "medium fluid" or simply "medium". A further suitable liquid to be used in accordance to the present disclosure can be a coolant aqueous solution such as for instance the TCS COOLANT supplied by TCS Micropumps^{™} Ltd. Such a liquid solution has further beneficial characteristics for the herein disclosed device as for example improvement of the contact at the interface between heat exchanger and coolant leading to increased thermal efficiency, inhibition or limitation of corrosion and/or algae and microbe growth, lubrication of pump seals (thus increasing pump life), reduction of air locks and so forth.

In the frame of the present disclosure, the expression "operatively connected" reflects a functional relationship between the several components of the haptic device or the entire system among them, that is, the term means that the components are correlated in a way to perform a designated function. The "designated function" can change depending on the different components involved in the connection; for instance, the designated function of a manifold operatively connected to a container is the regulation of the fluid medium flow between the container and a pipeline via the opening/closing of valves and/or activation/deactivation of a pump. Similarly, the designated function of a valve operatively connected to a display is the regulation of influx/efflux of the fluid medium through the display.

A "display unit" is a portion of the device or system that comprises a tactile display. A "tactile display", also referred to herein as "cell display" or "tactile cell", in the frame of the present disclosure refers to an output device for presentation of information in a tactile form. A tactile display is a user-device interface that can reproduce as closely as possible the tactile parameters of an object, either real or virtual, such as shape, surface texture, roughness, temperature and so forth. Particularly, a tactile display is an element that can provide pressure sensations, possibly combined with temperature sensations, to a user, including pinching, via fluid inflation or filling of a portion thereof; consequently, in preferred embodiments, a tactile display does not include devices such as motors, electro-magnetic actuators, vibration generators and the like.

A "Peltier element" is a thermoelectric device that uses the Peltier effect to create a heat flux between the junction of two different types of materials. Also known as Peltier cooler, heater, or thermoelectric heat pump, it is a solid-state active heat pump which transfers heat from one side of the device to the other, with consumption of electrical energy, depending on the direction of the current. It can be used either for heating or for cooling, although in practice the main application is cooling. It can also be used as a temperature controller that either heats or cools.

An "elastic material" is a solid material having the tendency to return to its original shape after being compressed, stretched, expanded or otherwise deformed. An elastic material is particularly suitable for the manufacturing of the tactile display of the device, since it could permit, enhance or ameliorate the range of possible user's feedbacks provided by the display in terms of tactile sensations. A non-exhaustive and non-limiting list of suitable elastic materials comprises polymeric materials such as silicone (for example polydimethylsiloxane PDMS), nitrile rubber, latex, polyurethane, polyisoprene (synthetic rubber), any kind of elastomers, the Tango family of rubber-like materials (for example TangoPlus or FullCure930) and the like.

The haptic device comprises one or more sensors for detecting and possibly storing at least a user's physiological parameter, an environmental parameter or a combination thereof, and is operatively connected with at least one element of the haptic device. A "sensor" as used herein is a device that detects (and possibly responds to) signals, stimuli or changes in quantitative and/or qualitative features of a given system, or the environment in general, and provides a corresponding output. The output is generally a signal that is converted to human-readable display at the sensor location or transmitted electronically over a network for reading or further processing. The specific input could be for instance light, heat, motion, moisture, pressure, or any one of a great number of other environmental phenomena. A sensor preferably comprises a means for detecting and possibly storing user's physiological parameter, an environmental parameter or a combination thereof. The sensor can therefore comprise a data storage device to hold information, process information, or both. Common used data storage devices include memory cards, disk drives, ROM cartridges, volatile and non-volatile RAMs, optical discs, hard disk drives, flash memories and the like. The information detected and collected by sensors can relate to a user's physiological parameter such as for instance muscle contraction (including postural muscle contraction), heart work rate, skin conductance (also called galvanic skin response GSR), respiratory rate, respiratory volume, body temperature, blood pressure, blood level of organic/inorganic compounds (e.g. glucose, electrolytes, amino acids, proteins, lipids etc.), electroencephalogram, sweating and so forth. Alternatively or additionally, the information detected and collected by the sensor can relate to environmental parameters such as temperature, humidity, light, sounds and the like.

Preferably, sensors further comprise means for transmitting the detected and possibly stored data concerning the above-mentioned parameters to a computer, and more preferably through a wireless connection. "Wireless" refers herein to the transfer of information signals between two or more devices that are not connected by an electrical conductor, that is, without using wires. Some common means of wirelessly transferring signals includes, without limitations, WiFi, bluetooth, magnetic, radio, telemetric, infrared, optical, ultrasonic connection and the like.

In one embodiment, sensors further comprise means for wirelessly receiving a feedback input from a computer able to regulate the functioning of the device. In one embodiment, sensors are operatively connected to a display unit and/or to a manifold. The main actuation unit controls the cells in the device without any cable (depending on the configuration, but at least in a configuration where the valves are on a main manifold). The main actuation unit could feature a printed circuit board (PCB) with e.g. a microcontroller controlling all the components (i.e. pumps, valves, sensors and any other component mounted on a main manifold). For this reason, the board manages the low-level functions such as a closed feedback loop controlling the pressure and possibly temperature in the cells. The board can be seen as a driver for the device communicating wirelessly with a computer or a mobile phone managing high-level functions.

The expression "haptic profile" is herein used to intend the sequence of instructions necessary to functionally operate a haptic device according to one or more input data. More precisely, a "haptic profile" refers herein to the instructions encoding an activation, such as a spatio-temporal activation, of a plurality of operatively connected tactile displays in a display unit, the activation being coherent with the audio profile of an audio file. A haptic profile encodes the activation pattern of a display unit based on the type of sensory tactile to be provided (i.e. pressure, possibly and in some embodiments preferably combined with temperature), the number and identity of displays and their relative positioning, the duration of stimulation of the displays, the synchrony/asynchrony of a stimulus onset among the displays and the percentage of activation overlap between the displays in a unit; at the same time, the modulation of those several parameters is tightly linked to the output of an audio processing (referred to herein as "audio profile"), which in turn gives rise to a haptic profile via an audio-to-haptic conversion, in which an elaboration of processed frequencies and amplitudes of an audio signal produces an audio profile and therefore a precise signature of tactile display activation.

For "computer-readable data carrier" or "computer-readable medium" is herein meant any available medium that can be accessed by a processor and may include both a volatile and non-volatile medium, a removable and non-removable medium, a communication medium, and a storage medium. A communication medium may include computer readable instructions, data structures, program modules or other data in a modulated data signal such as a carrier wave or other transport mechanism, and may include any other form of an information delivery medium known in the art. A storage medium may include RAM, flash memory, ROM, erasable programmable read-only memory ("EPROM"), electrically erasable programmable read-only memory ("EEPROM"), registers, hard disk, a removable disk, a compact disk read-only memory ("CD-ROM"), or any other form of a data storage medium known in the art.

One aspect of the present invention relates to computer-implemented method for operating a haptic device, and a computer-readable medium having computer instruction code recorded thereon, the instruction code configured to perform a method when executed on a computer operatively associated with a haptic device, the haptic device comprising a plurality of tactile or thermo-tactile displays configured to provide haptic stimuli to a user, the method comprising the steps of:
a) processing an audio signal derived from an audio file, thereby obtaining at least one profile of frequencies and amplitudes of the audio signal (i.e. an audio profile);
b) converting the frequencies and amplitudes profile into a haptic profile; and
c) operating the haptic device according to the haptic profile.

Another aspect of the present invention relates to a multisensory system comprising:
a) a haptic device comprising a plurality of tactile displays configured to provide haptic stimuli to a user;
b) a data processing apparatus operatively connected with the haptic device, the apparatus comprising a processor configured to perform the computer-implemented method as described herein; and
c) at least one audio device configured to reproduce an audio file.

Embodiments of the haptic device according to some aspects of the present invention will be described hereinafter in more details. However, in one preferred embodiment, the haptic device is the one described in U.S. Patent No. 9,703,381 B2, owned by the present applicant. This kind of haptic device is particularly suitable for the target applications of the system, as will be detailed later on.

A haptic device according some aspects of the present invention is flexible and adaptable to the user's anatomy, and can even, in one embodiment, provide thermo-tactile haptic feedbacks. Generally, the device comprises at least an actuation unit connected to a flexible display unit. The actuation unit pneumatically and/or hydraulically controls the pressure and possibly temperature of a fluid medium, such as liquid or a gas, to provide tactile, and possibly at the same time even temperature, cues, to the user touching the display. The tactile cues are created by controlling the shape of a flexible membrane of the display through the fluid medium pressure. In one embodiment, contrary to known tactile displays that make use of several rigid actuators in order to obtain a multimodal feedback, a haptic device features one single actuation system that generates the integrity of the multiple haptic feedbacks, both tactile and proprioceptive (e.g. thermal cues).

In one embodiment, thermal cues are provided by the heat exchange between the fluid medium and the user's skin through the same membrane. The temperature of the fluid medium stream flowing in the display is achieved by mixing several fluid streams at specific temperatures. These fluids are heated or cooled to specific temperatures using e.g. Peltier elements, and can be stored in buffer tanks. The medium flow and pressure are controlled using a combination of micro pumps and valves. The flexible display is composed of an array of cells herein named tactile displays or cells. The number of cells and their disposition is modular in order to adapt the cell density to the type and surface of skin. In one embodiment, the medium pressure and temperature can be controlled in each individual display cell using a system of valves and manifolds, possibly embeddable in a portable actuation unit. The tactile display can have different functional shapes adapted to the user's anatomy.

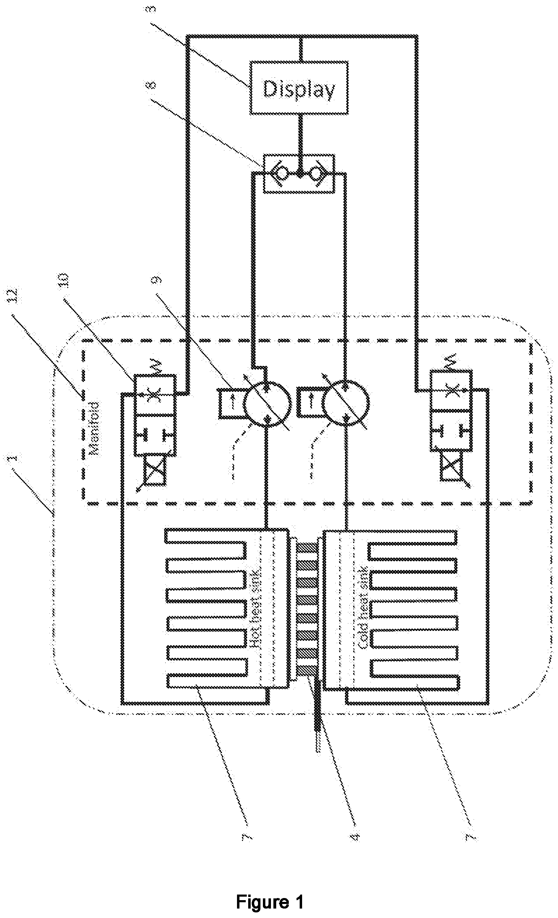

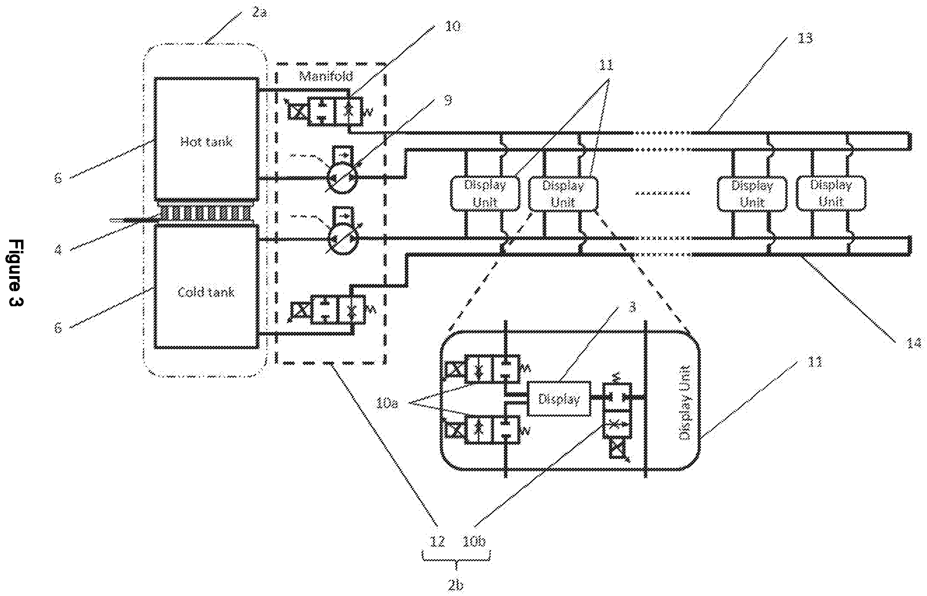

With reference to Figures 1 and 3, in one exemplary embodiment, the device is divided in two parts: an actuation unit 1 comprising a pressure regulation unit 2b, and the display 3. In an additional embodiment, the actuation unit 1 further comprises a thermal conditioning unit 2a. The pressure regulation unit 2b controls the pressure of the medium flowing through the display 3 (for each unit) working as an interface with the user's skin. A thermo-tactile feedback is achieved by using the actuation unit 1 to control the temperature of one or several fluid medium streams.

The thermal conditioning unit 2a uses one or several Peltier element(s) 4 to contemporaneously heat the fluid medium (liquid or gas) flowing on one surface while simultaneously cooling the medium flowing on its opposite surface. By doing so, the efficiency of the Peltier elements 4 is greatly increased compared to conventional applications when only one side of the Peltier elements 4 is used for thermal stimulation, while the other is forced to a constant temperature thus wasting power. Several Peltier elements 4 can be used in series to, in turn, cool down or warm up two fluid medium streams. In one embodiment, they can also be used in parallel thus creating several medium streams at different temperatures. The temperature of the fluid medium entering the display 3 is achieved by mixing streams of the medium at different temperatures. The temperature range of the device is set by the coldest and warmest stream.

In one embodiment, tanks 6 can be used to store the medium at different temperatures. These temperatures delimit the thermal working span of the device and are customizable. Tanks 6 work also as buffers compensating for the change in temperature resulting from the medium returning from the display 3.

The heat transfer between the Peltier element(s) 4 and the fluid medium is achieved through heat conduction and natural or forced convection. Furthermore, heat sinks 7 (typically made of copper and aluminium) can be used to increase heat exchange. Accordingly, in one embodiment of the invention, the Peltier element(s) 4 can be interposed in between two tanks, thus heating and cooling the medium in both tanks through natural convection. In this configuration, the medium in the tanks can also be set into motion using a mixing system, thus achieving forced convection.

In another embodiment, the Peltier element(s) 4 can be interposed in between two heat sinks and the medium pumped through the the heat sinks to achieve forced convection.

In one embodiment, the fluid medium is pumped through the heat sinks 7 and the heat transferred from the Peltier element(s) 4 is sufficient to rapidly reach the target medium temperature, both on the hot and cold side. In this configuration no tanks 6 are required (instantaneous heating and cooling).

The desired display 3 temperature is achieved by mixing various medium streams at different temperatures into a mixing chamber 8. However, in an alternative embodiment, this mixing can also take place directly inside the display 3. The temperature of the mixed fluid medium is regulated by controlling the proportions of hot and cold medium injected into the chamber of the display unit 3 using the pumps 9, the opening of the valves 10 or a combination of both. The temperature feedback is provided using temperature sensors in the display. Small sensors with low thermal inertia such as thermocouples, thermistors, thermal resistances and the like are introduced inside the display through the pipelines.

The pressure regulation unit 2b can control the pressure in the display by using two components: the pump(s) to generate pressure and the valve(s), called outlet valves 10, placed at the outflow of the display(s) 3 to control the medium flow. By controlling the pressure applied by the pumps 9 and/or the valve 10 a precise control of the pressure in the display 3 is achieved. Pressure sensors can be placed after the inlet valves to provide pressure feedback at the display(s).

In a preferred embodiment, the entire system is completely filled with a medium, sealed and air-tight. By doing so, when a pump, for example the pump in the hot tank in a configuration with two tanks, takes medium out of a tank, the tank pressure drops. After the medium goes through the system and out of the display 3, it will be forced to return to the hot tank due to the pressure difference. If cold and hot medium is pumped from both tanks but in different proportions, for example a larger volume of hot medium than cold medium is pumped as the desired display temperature is rising, the mixed medium exiting the display will be divided between the two tanks according to the same proportions due to the different pressure drops in the two tanks. Thus, by sealing the system it is ensured that both tanks remain full at all times, and in any position. In addition, by having the same proportions of medium returning to the corresponding tanks, the impact of the returning mixed medium on the tanks thermal equilibrium will be minimal, thus reducing the power consumption of the system.

Figure 1 shows an exemplary schematic representation of an embodiment of the system combining two previous embodiments. In this system, no tanks 6 are used and the medium goes through a hot and cold heat sink sandwiching a Peltier element 4. The power of the Peltier element 4 is sufficient to bring the medium to the desired temperature almost instantaneously. The two medium streams are then mixed in the mixing chamber 8 connected to the display 3. The medium leaving the display consequently goes back to the appropriate tanks through two valves 10 mounted on the main manifold 12 (itself mounted on the tanks 6) and that are used to control the pressure in the display 3.

In a preferred embodiment, a display 3 includes a main body 16 comprising in it one or several hollow chambers 17. This body can be made of rigid material (plastic, metal, etc.) or of a flexible polymeric material (e.g. PDMS). A thin elastic membrane 18 is attached on the main body 16, covering the hollow chamber(s) 17. The membrane 18 can be made out of any kind of soft and stretchable polymer, such as a silicones or elastomeric materials. The material and thickness of the membrane 18 is selected based on the quantity of heat to be transferred and the desired elasticity, in order to obtain a convenient deformation of the membrane. The combination of a display hollow chamber 17 with the membrane 18 covering it is called a display cell 19.

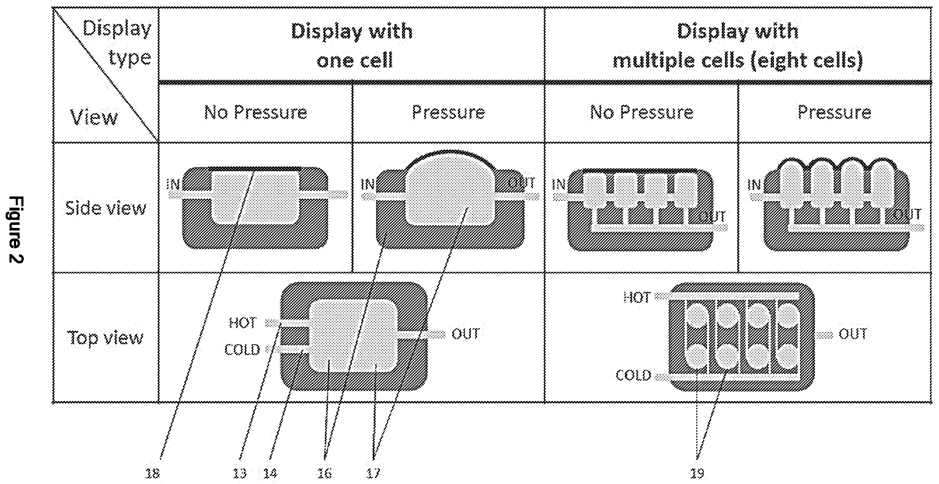

Figure 2 presents an exemplary schematic sketch of two display types 3 (one with a single display cell 19, the other with multiple cells 19), in the pressured and resting states. The display cells 19 size can vary depending on the tactile resolution of the skin stimulated (i.e. feet, glutei or back) and the desired type of stimulus. When the fluid medium flows through the chamber, heat exchange will occur between the user's skin touching the membrane 18 and the medium. By building up the pressure inside the display cell 19, the membrane 18 will fill or inflate, thus providing a tactile feedback.

Figure 3 shows an exemplary schematic view of one embodiment of the device. It features two tanks 6, one comprising a cold fluid medium and one comprising a hot fluid medium, with a Peltier element 4 interlaid between both tanks 6. A main manifold 12 comprising two pumps 9 and two valves 10 are mounted on the tanks 6. The two pumps 9 move the medium from the hot and cold tank into two pipes respectively leaving the manifold 12. These two pipes, named the hot main line 13 and cold main line 14, bring the hot and cold medium close to the area(s) that need(s) to be stimulated. The mediums from the hot and cold lines 13, 14 then flow back to the hot and cold tanks 6 through the manifold valves. These flows in and out of the tanks 6 generate movement of the medium in the tanks 6 and thus forced heat convection between the Peltier element 4 and the medium 5, thus increasing the efficiency of the system. By storing hot and cold medium 5 in tanks 6 and mixing them to obtain the desired temperature at the display 3, extremely high rates of temperature change can be achieved.

Display units 3 can be attached anywhere along the cold and hot lines 13, 14. A display unit 11 consists in a display 3 and three valves 10 (solenoid or proportional). The display unit 11 unit has two inputs, from the hot and cold line respectively. Two inlet valves 10a are used to control the amount of the hot and cold medium entering the display. The mixing of the two medium takes place directly in the display unit 3. Finally, the outlet valve 10b is used to control the pressure inside the chamber 17 as previously explained. When the temperature and the pressure in the display cell correspond to the desired level both the inlet and outlet valves are closed, thus isolating the display unit from the hot and cold main lines.

The cell resolution of this display is scalable to any size and density. As will be apparent for a person skilled in the art, the same actuation unit will control any shape of display, and the display shape can be easily adapted to the function.

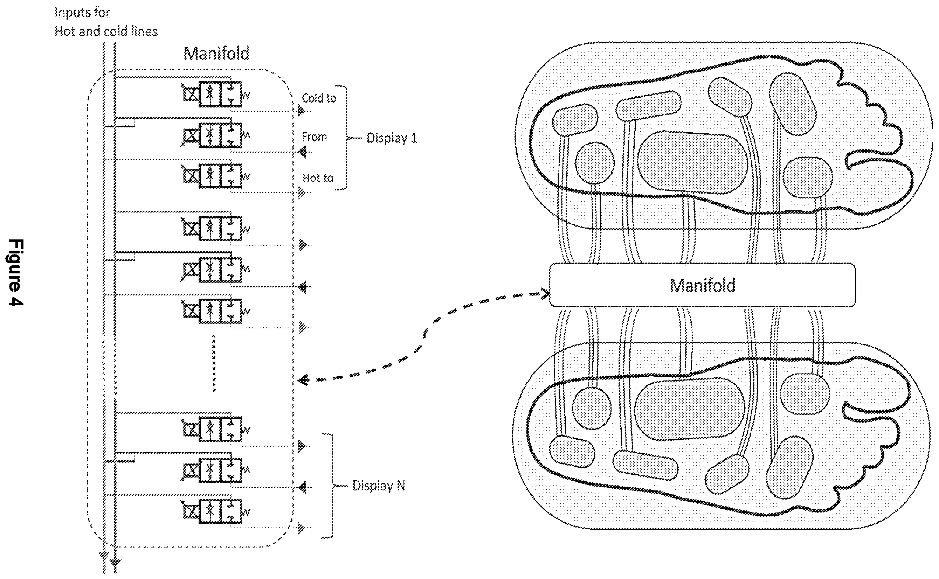

Figure 4 shows an exemplary embodiment of the device, according to some aspects of the present invention. In this scenario two displays, each having seven cells, are placed under a user's feet. In this configuration, the inlet 10a and outlet valves 10b regulating the pressure and flow in each display cells are not placed directly under the feet but rather located remotely on a manifold 15. This manifold is connected to the hot and cold main lines. In the shown embodiment, three connections for each cell (2 outlets for the hot and cold medium and 1 inlet for the mixed return medium) are present, as well as three valves per cell. The number of cells can vary depending on the number of stimulation points desired.

In another embodiment of the device, all the valves from the displays (3 valves per display) are placed on the main manifold 12 over the tanks 6. By doing so there are only pipes going out of the main control/actuation unit and no valves or other manifolds have to be placed elsewhere along the device. On the other hand, there will be three pipes connected to each display. This solution is ideal when a smaller number of display is required as it reduces the system complexity.

The placement of the valves 10 is flexible and can be changed depending on the application. In another embodiment of the device, the inlet valves 10a are placed next to their display 3, but the outlet valve 10b is placed in the main manifold over the tanks. This is a hybrid embodiment combining two previous designs. Thus, depending on the application and the complexity of the stimulation to be performed, the system can be centralized in one main actuation unit or decentralized over several light manifold placed at the area(s) of interest.

In another embodiment of the device four pumps are used instead of two. There are two pumps per tank, one is placed upstream, the other downstream. The pump downstream must be a self-priming pump. By doing so, a precise control of the pressure in the display during medium flow is achieved. In fact, with only one pump pushing, the pressure in the display during the flowing phase is dependent on the hydraulic load following the display while with one pump pushing and the other sucking the pressure in the display during the flowing phase is controllable.

In one embodiment, the display cells, the sensors, the valves, the pumps and the piping are combined into one flexible polymer "skin". This is done by moulding polymers such as polydimethylsiloxane (PDMS) or by 3D printing the display using rapid prototyping machines with flexible materials (i.e. Tango Black Plus). Using these production methods the display can be fabricated with functional shapes and also easily embedded into articles of manufacture such as mats, pillows, pieces of furniture, chairs, deck-chairs or vehicles' seats, as well as into a wearable device or garments. Printed electronics using functional inks could be used to integrate the sensing directly in the membrane of the cells. For the temperature sensing, a thermocouple is created by printing two lines of different semiconductors that overlay each other in one point. This thermocouple is ideally placed because it is in contact with the skin. It is flexible and does not get damaged by bending the device. Further, in addition to the sensors, the wires going from the control/actuation unit to the display(s) can be printed, thus removing free wires from the system. Deformation sensors can be produced by printing strain gauges on the edges of the display membrane. Finally, the valves and/or the pumps can even be microvalves and micropumps, and can be molded or printed as part of this "skin". Those elements can be substantially made of an elastic polymeric material, possibly manufactured through microfluidic manufacturing techniques (e.g. photolithography, polymers molding techniques and the like).

The present device provides multiple tactile feedbacks as well as force feedback that can be displayed individually or simultaneously:
1) The pressure control system is used to control the stiffness of the display membrane. By building up pressure, the display becomes stiffer and vice versa. A dynamic control of the pressure can be also provided, in order to create e.g. a pulse feedback. This is done by building up the pressure in the chamber, thus inflating the membrane, and subsequently releasing the pressure.
2) In an additional or alternative embodiment, by driving the pumps in the opposite direction while keeping the outlet valves 10b closed, suction at the level of the display membrane is generated, thus providing a suction feedback.
3) In an additional or alternative embodiment, by using a main body 16 with lower rigidity, it is possible to combine the orthogonal deformation of the membrane with a tangential deformation of the main body. This will provide an additional tactile feedback, i.e. a lateral skin stretch.
4) In an additional or alternative embodiment, the membrane has a high porosity. This in turn increases the humidity under the user's skin. By applying pressure, or suction, it is possible to control the humidity, thus providing humidity feedback.
5) In an additional or alternative embodiment, the display has a high density of small tactile cells used to generate a texture under the user's skin. In a passive version, the user's skin is moving on the display and the user can feel the edges due to the inflated cells. In the active version, the user's skin is immobile on the display and the cells are activated in specific spatiotemporal patterns giving the illusion of stroking a specific texture.
6) In an additional or alternative embodiment, force feedback can be achieved at any point of the device by e.g. building up the pressure in the lines instead that in the displays. By closing all the inlet and outlet valves of the displays and the outlet valves 10b in the main manifold, the pumps will build up pressure in the pipes, rigidifying them. Relief and bypass valves can be used to keep a specific area of the pipe line under pressure while releasing pressure from the rest of the line, thus focusing the force feedback on that specific area.
7) In an additional or alternative embodiment, the inlet valves 10a are solenoid valves driven with an adjustable pulse width modulation (PWM) frequency. The fast switching of the valve generates a vibration that moves along the pipe. If the display 3 is placed in vicinity of the valve, this vibration is transmitted to the membrane, thus creating vibration feedback under the skin. This fast switching frequency can be in the range of 220Hz, the frequency at which the skin mechanoreceptors (FA) have the strongest response. When the vibration feedback is not required, the inlet valves can simply be used as ON/OFF valves. The control of the temperature in the display with the valves 10 does not require high frequency shifting, for example the hot medium inlet valve can stay open until the display medium heats up to the desired temperature and be closed afterwards.
8) In an additional or alternative embodiment, a pinching force feedback is generated by connecting a third display cell interposed between two cells. This new middle cell, herewith named stiffness cell, can have the same design as previously described cells, with a main body and a membrane. Alternatively, it can have a main body with two membranes, one on top of the main body and the other on its bottom. It can also have only two membranes attached to each other, thus making a balloon. This chamber can be filled with the fluid medium and its stiffness, similarly to the stiffness feedback applied on the individual fingertips, can be changed by modifying the pressure of the medium inside the chamber. The stiffness of the cell, as well as the flexibility of its membranes, depends on the displacement required for the pinching movement and the forces required.

Having described in detail the device, the multisensory system further features a data processing apparatus operatively connected with the haptic device, the apparatus comprising a data processor configured to perform a computer-implemented method for operating the haptic device, according to another aspect of the present invention. The data processing apparatus can be any suitable device such as computers, smartphones, tablets, data processing units, servers, voice-activated devices (i.e. smart speakers/voice assistants) and the like.

In one embodiment, the apparatus comprises memory storing software modules having computer-executable code that provide functionality when executed by the processor. The modules include an operating system that provides operating system functionality for the apparatus. The modules further include a haptic conversion module that converts an audio signal into a haptic profile which encodes information on how to operate the haptic device, as disclosed in more detail below. The apparatus, in embodiments that transmit and/or receive data from remote sources, further includes a communication device, such as a network interface card, to provide mobile wireless communication, such as Bluetooth, infrared, radio, Wi-Fi, cellular network, or other next-generation wireless-data network communication. In other embodiments, communication device provides a wired network connection, such as an Ethernet connection or a modem.

The computer apparatus executes a first step of the method by processing an audio signal derived from an audio file so to obtain at least one profile of frequencies and amplitudes of the audio signal. According to one embodiment, an envelope of the audio signal is first extracted. An envelope can be extracted using all frequencies of an original audio signal or a filtered version of the original audio signal. However, the envelope itself does not have the same frequency content as the original audio signal.

In some embodiments, to obtain a profile of frequencies and amplitudes of the audio signal the processing comprises applying a bandpass filter on the input audio file, broadly centred around a desired frequency. The signal is then rectified, and a low-pass filter is applied to obtain the envelope of the signal. In some embodiments, the envelope is then down-sampled, and a noise threshold amplitude is applied. In some embodiments, a window function such as a convolved Hanning window, which time constant is based on prior knowledge on the desired signal, is then applied.

Once obtained a filtered envelope, the computer apparatus executes a second step of the method by converting the frequencies and amplitudes profile in the form of a filtered envelope into a haptic profile by a haptic converter module. As the amplitude of the envelope encodes the "strength" of an audio signal, which will be later on converted into the "strength" of a haptic feedback, the peaks and the valleys of the envelope (i.e. its local maxima and minima) are tracked. Prior knowledge on the type of the desired haptic feedback can also be used to fine tune the peak research by defining minimum times between peaks and minimum peak amplitudes.

Once the peaks and valleys located, the computer apparatus executes a further step of the method by operating the haptic device according to the obtained haptic profile. By "operating the haptic device according to the obtained haptic profile" is herein meant that the processor transmits a signal associated with the obtained haptic profile to the haptic device, which in turn outputs haptic sensations to a user. In particular, actuation unit 1 is configured to receive the information encoded by a haptic profile, and configured to operate the pressure regulation unit 2b and possibly the thermal conditioning unit 2a in order to provide tactile, possibly thermo-tactile, feedbacks to a user via the tactile display 3.

In some embodiments, the multisensory system further includes at least one audio device configured to reproduce an audio file. The device can be for instance one or more speakers. The computer's processor may transmit an audio signal to speakers, which in turn outputs audio effects. Alternatively, earphones or headphones could be used. In some additional embodiments, the multisensory system further includes at least one video device configured to reproduce a video file. In some additional embodiments, the multisensory system further includes at least one smell device configured to provide smells to a user.

Moreover, one aspect of the present invention relates to the use of the multisensory system as described herein for Yoga training, meditation, massages or hypnosis' induction. In this context, another aspect of the present invention relates to a method for performing Yoga training, meditation, massages or hypnosis' induction, the method comprising the steps of:
a) allowing a user to contact the haptic device of the multisensory system;
b) reproducing an audio file via the audio device of the multisensory system; and
c) operating the haptic device of the multisensory system according to the method.

In one embodiment, the reproduced audio file is the same used for obtaining a haptic profile.

To describe and illustrate more clearly the aspects of the present invention, the following examples are provided in detail, representing different embodiments, which however are not intended to be limiting of the scope of invention as described in the appended claims.

### EXAMPLES

### Example 1 - Yoga training and meditation

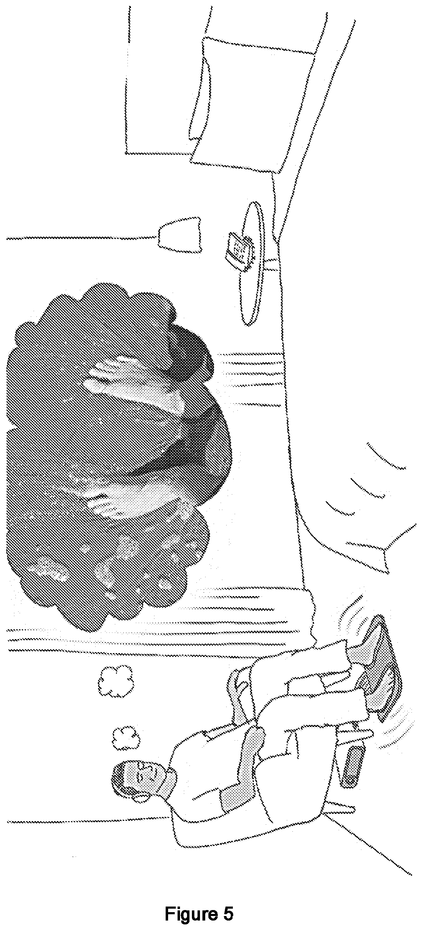

In the frame of Yoga training and meditation, a real world embodiment of the multisensory system has been conceived and implemented, wherein a multimodal haptic device is adapted for the feet (Figure 5). The practitioner simply sits down, locates the feet on the haptic interface, wears a pair of headphones, closes his eyes and starts the meditation session. The practitioner is first introduced into the practice by encouraging him to be aware of his body, and breath, the two most classical anchor point of contemplative practices. Then, the practitioner is asked to visualize himself "sitting on a deck or peer on the shore of a lake" and having his "...feet hang over the deck and touching water".

Practitioner starts experiencing actual sensations of water waves coming to their feet. Custom-made algorithms tailored for creating a haptic profile allow to automatically match the dynamic pressure and temperature changes provided through the haptic device with a pre-recorded audio of water waves to create a holistic water experience. The guidance encourages the practitioner to pay attention to the bodily sensations that are now materialized through temperature and pressure modulations.

Through computer-, app-, or voiced-based interfaces, the user is able to select a preferred scenario (e.g. a calm day on the Indian Ocean or a windy day on the Pacific), and fine-tune the experience by choosing the temperature of the water and the sand or the strength of the waves. This ultimate personalization of the scenario is only possible with a precise control of the tactile stimuli and their coordination with sound. This personalization can be fully controlled by the user or automated through machine learning techniques. Other naturalistic scenarios might include sounds such as water movements (e.g. drops, etc.), the sound of fire, the sound of snow dropping or being trampled, the sound of mud being trampled, the sound of leaf or other naturalistic or non-naturalistic elements. The audio file including sounds from naturalistic scenario can be pre- or online recorded and merged with a pre- or online recorded guidance. For example, the user might follow a live meditation guidance in a room, encouraging to pay attention to water waves coming to the participant's feet, (not physically present in the room but) rendered through the multisensory haptic device. In this case, the pre-recorded sound of water waves is merged with the online-guidance (captured by artificial intelligence Al, voice-based, interfaces connected to the multisensory haptic system) to create a meditation experience that blends virtual and real elements (the pre-recorded waves are synchronized with the real-time voice, or their temperature is set based on the instructions of the live guiding voice, e.g. "now focus on the warm waves").

Other versions of the multisensory device might also include a smell unit, controlled by the control processing unit to further enrich the multisensory experience as well as include the monitoring of physiological signals such as breathing, heartbeat, skin conductance, etc. These bio-signals can be synchronized through the multisensory control unit with the naturalist sound (and its related thermo-tactile profile), and the guidance. The multimodal haptic device can be integrated in a Yoga mat, a pillow, a piece of furniture (e.g. chair, bed, armchair, etc), the seat of a vehicle, a wearable device or a garment.

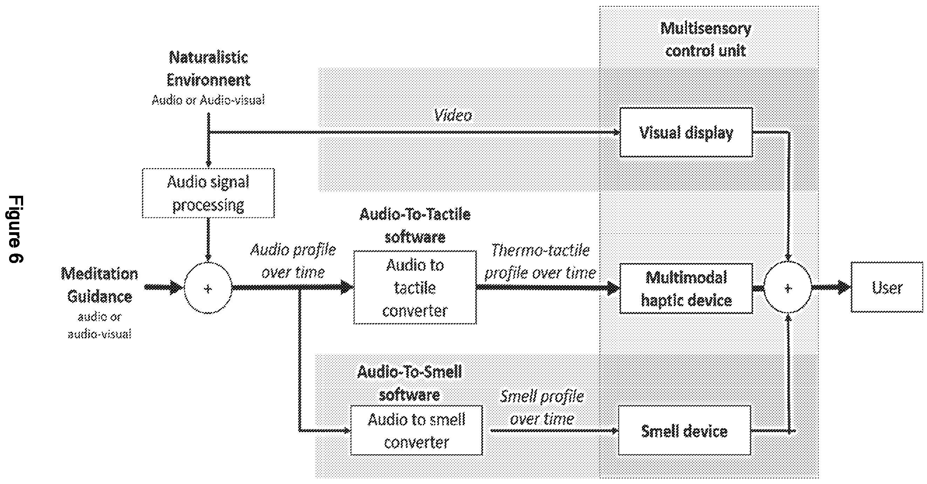

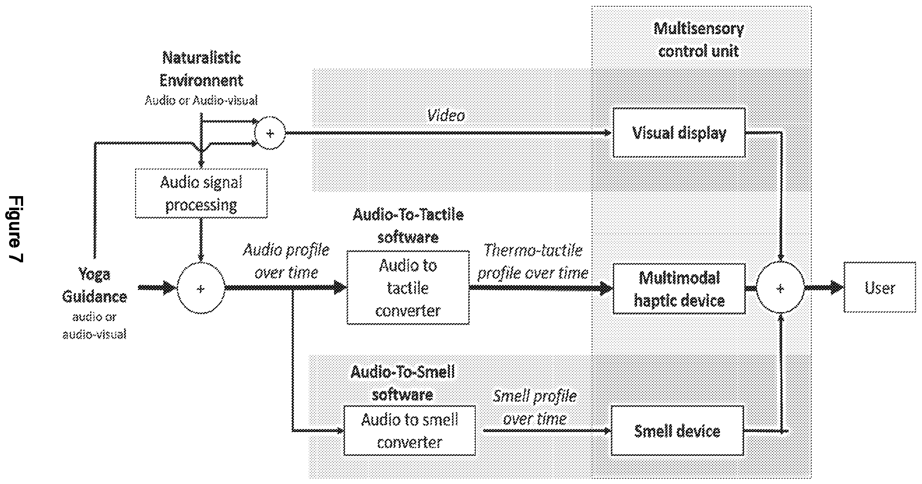

Figure 6 shows one embodiment of the different elements that empower the meditation and guidance, by matching the guided description of the sensory stimulation occurring on the practitioner's body to its multisensory counterparts, according to an aspect of the present invention. Similarly to Figure 6, Figure 7 shows one embodiment of the components of the system that empower Yoga guidance. The only difference is that in the case of Yoga, the guidance, i.e. the different Yoga poses, can also be seen by the practitioners.

### Example 2 - Sound To Haptics: the case of waves

In this embodiment a sound of waves is converted into a tactile stimulation provided with a thermo-tactile display composed of 3 cells (each cell is controllable in pressure and temperature).

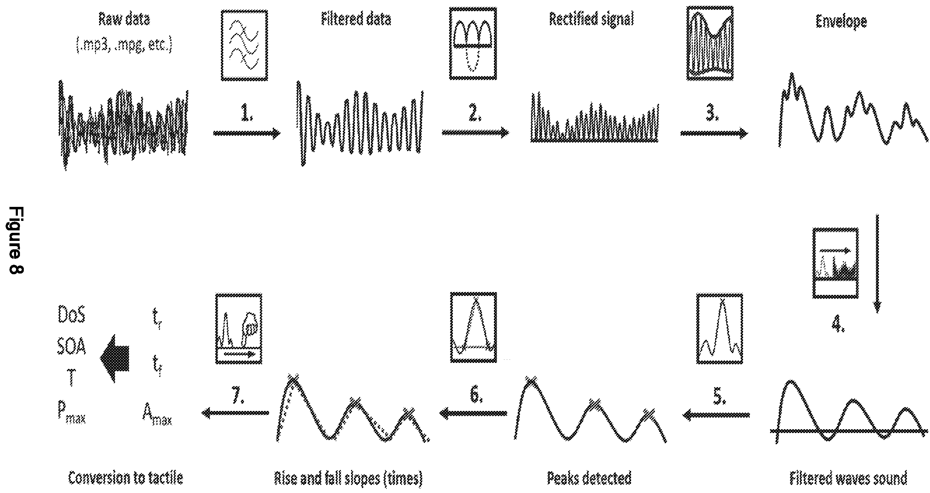

First, the algorithm filters the input audio file provided by the user and detects the desired water movements following the steps shown on Figure 8. The algorithm starts by applying a bandpass filter 1 on the input audio, broadly centered around the frequency of the desired water movement sound (e.g. waves), in order to eliminate undesired sounds with significantly different frequencies (e.g. a seagull sounds in an ocean scenario).

The signal is then rectified 2, and a low-pass filter is applied to obtain the envelope 3 of the signal. The envelope is then down-sampled to accelerate the execution time of the following steps and a noise threshold amplitude is applied in order to filter out non desired sounds with a similar frequency range as the waves.

A convolved Hanning window 4, which time constant is based on prior knowledge on the desired signal (e.g. it usually takes ocean waves more than 1 sec to move in and recede), is then applied to precisely detect the waves. Prior knowledge on the shape of the signal (e.g. an ocean wave moves in fast and recedes slowly) can be used to tune the convolution window and obtain even more precise results.

As the amplitude of the envelope now encodes the "strength" of the water movement (its flow), the peaks of the envelope 5 are tracked or, in other words, the envelope's local maxima. Prior knowledge on the type of desired water movement (type of wave or stream of water) can also be used to fine tune the peak research by defining minimum times between peaks and minimum peak amplitudes.

Once the peaks located, it is possible to estimate the onset of the wave, i.e. the last occurrence before the peak when the amplitude of the envelope goes over the noise threshold, and the end of the wave, i.e. the first occurrence after the peak when the amplitude of the envelope falls under the noise threshold. From there, the rise and fall time of the waves are also derived and the wave is fully characterized. In order to convert the waves into a tactile stimulation spread over three cells, three waves' parameters are considered: the rise time, the fall time, and maximum amplitude of the wave.

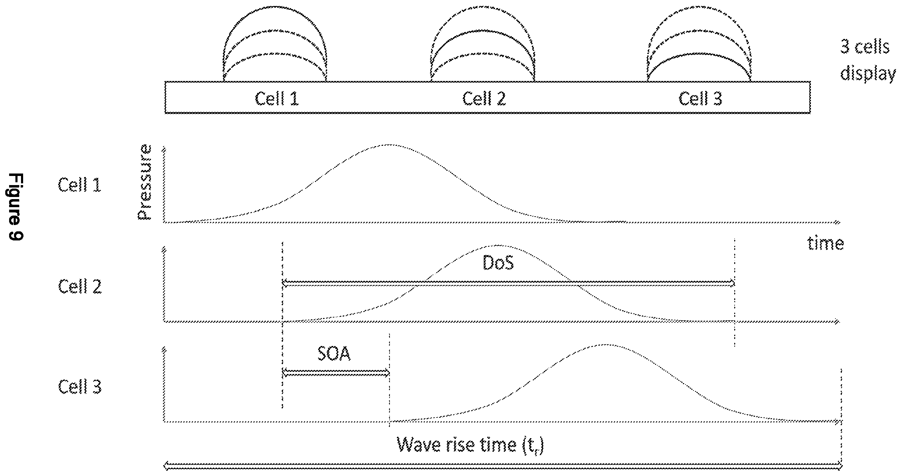

To give the sensation that the wave is moving on the user's skin, the rise and fall time of the wave are encoded as a spatio-temporal pattern of activation of the three (3) display cells (Figure 9), thus resulting in two distinct tactile patterns for the rise and fall of the wave. To ensure that the user perceives a fluent tactile movement encoding a wave, and not three discrete stimulations, the activation of the cells overlap in time. The overlap is determined by the body part stimulated (because of the different spatial resolutions in different body parts) and by the distance between the display's cells. For a given rise (and fall) time of the wave (tr or tf), the duration of stimulation of the cells (DoS) and the stimulus-onset asynchrony (SOA) are determined based on the percentage of overlap (Op) with the following equations:
$tr = 3 \times DoS \times Op$ (1)
$SOA = \left(1-Op\right) \times DoS$ (2)

The maximum amplitude of the wave is normalized by a maximum tactile pressure value (determined by the user or the maximum pressure available in the display) and used as input pressure command for the cells.

Finally, the temperature of the cells is determined by the state of the waves. When the waves are moving in the temperature is the coldest, once they recede the temperature is still cold but slightly warmer and finally the temperature in between waves rises up to emulate the warmer ground (e.g. the sand in the ocean scenario). The temperature range can be selected by the user to optimize comfort. Furthermore, the temperature can also be modified by other audio events, for example the water can get colder if the wind rises, or if the rain starts pouring in.

### Example 3 - Behavioural experiment In a meditation setting

In a behavioural experiments with 30 participants without any previous meditation experience, we tested whether the described multisensory scenario (water waves simulated through both touch and sound) can increase the immersion of the meditation (e.g., the sensations of being located on a lake shore and being touched by water waves) and improve the meditation experience (e.g. increased focus and absorption into the meditation). Participants were asked to perform three (3) experimental conditions: 1) in one condition they were asked to focus only on their breath; 2) in another condition they were exposed to a guided meditation scenario as the one described above, with water waves rendered through sound only; 3) in another condition they were exposed to a guided meditation scenario - as in point 2 - but with water waves that were simulated through sound and touch. Conditions were counterbalanced across participants and each meditation lasted approximately ten (10) minutes. To assess the meditation experience, after each meditation participants were given the following questionnaire and were asked to rate each question (Q) on a 0-6 Liker-scale (0, strong disagreement; 6, strong agreement):
Physical/body sensations:
   1. During the meditation, I felt pleasant sensations such as feeling relaxed, at peace, calm.
   2. During the meditation, I felt unpleasant sensations such as feeling restless, irritated, displeased.
   3. During the meditation, I felt as if my body was located on a lakeshore (Presence at Lake).
   4. During the meditation, I felt waves move gently over my feet (Feet in Lake).
   5. During the meditation, I felt as if the waves were touching my shoulders (Control).
Thoughts and other subjective impression:
   6. During the meditation, I was distracted (Distractedness).
   7. During the meditation, I felt that my mind was wandering or that I was day dreaming.
   8. During the meditation, I caught myself thinking of things I have to do later (in the future).
   9. During the meditation, I was aware of the different thoughts coming and going on my mind.
   10. During the meditation, I felt as if my thoughts were turning into sounds (Control).
Absorption in the meditation:
   11. During the meditation, I felt fully absorbed in the session (Absorbed into Session).
   12. During the meditation, the voice guiding me helped me to focus (Conditions Aided in Focus).
   13. During the meditation, I felt as if time was going by more quickly.
   14. During the meditation, I felt as if time was going by more slowly.
   15. During the meditation, I felt as if the guiding voice was moving further away (Control).

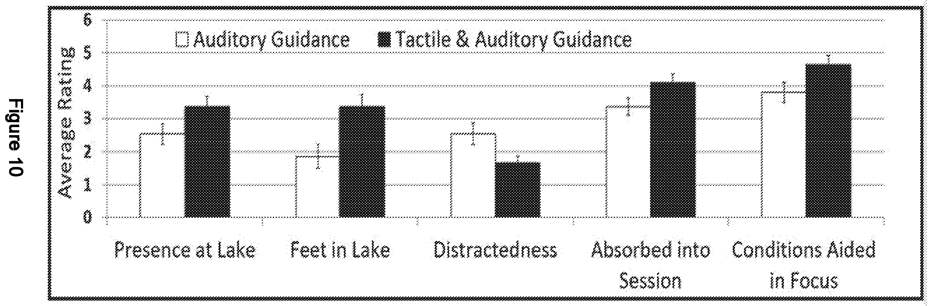

Results show that when comparing average ratings for the questionnaire answers, combined tactile and auditory guidance compared to auditory guidance alone increases physical realism of visualization (higher ratings for Q3 and Q4), absorption intro the meditation (Q11), and focus (Q12), and decreases distractedness (Q6). N=30, p<0.05 by paired Wilcoxon test, error bars by SEM. Control questions showed no significant modulation across two guidance conditions (Figure 10).

Importantly, increased realism (assessed through Q3) correlates with increased focus (Q12) and decreased distractedness (Q6). More specifically, difference in ratings between auditory plus tactile stimulation and auditory only for the Presence at Lake item (Q4) positively correlates to level of focus (Q12 ; rho = 0.476, p <0.05 by Spearman's test) and negatively correlates to distractedness (Q6 ; rho = -0.44, p <0.05 by Spearman's test). This suggests that the more participants were immersed in the naturalistic scenario (thanks to the combined tactile and auditory stimulation) the more they were engaged in the meditation.

## Claims

1. A computer-implemented method for operating a haptic device to provide the sensation that a wave is moving on the user's skin, the haptic device comprising a plurality of tactile display cells configured to provide haptic stimuli to a user, the method comprising the steps of:
a) processing an audio signal derived from an input audio file of water waves, thereby obtaining at least one profile of frequencies and amplitudes of the audio signal;
b) converting the frequencies and amplitudes profiles into a haptic profile; and
c) operating the haptic device according to the haptic profile to provide the sensation that a wave is moving on the skin of a user
wherein
- step a) comprises extracting an envelope of the audio signal; and determining the local maxima and minima of the envelope, thereby deriving the rise and fall time of the wave.

2. The method of claim 1, wherein step c) comprises activating the tactile display cells in a spatio-temporal fashion so that a maximum amplitude of the wave is normalized by a maximum tactile pressure value.

3. The method of claim 1 or 2, wherein step a) comprises applying a bandpass filter on the input audio file, broadly centered around a desired frequency; rectifying said signal; and applying a low-pass filter.

4. The method of any previous claim, wherein step a) comprises applying a window function such as a convolved Hanning window.

5. The method of any previous claim, wherein step c) further comprises providing thermal stimuli to a user via the thermo-tactile display cells of the haptic device.

6. The method of any previous claim, wherein the haptic device comprises 3 cells, and for a given rise time of the wave tr, the duration of stimulation of the cells DoS and the stimulus-onset asynchrony SOA are determined based on the percentage of overlap Op with the following equations: $tr = 3 \times DoS \times Op$ (1) $SOA = \left(1-Op\right) \times DoS .$ (2)

7. A multisensory system comprising:
a) a haptic device comprising a plurality of tactile display cells configured to provide haptic stimuli to a user;
b) a data processing apparatus operatively connected with the haptic device, the apparatus comprising a processor configured to perform the method of claims 1 to 6; and
c) at least one audio device configured to reproduce an audio file.

8. The multisensory system of claim 7, further comprising d) a computer-readable storage medium comprising at least one audio file.

9. The multisensory system of claim 7 or 8, wherein the haptic stimuli are force and/or pressure.

10. The multisensory system of any one of claims 7 to 9, wherein the tactile displays are pneumatically or hydraulically operated.

11. The multisensory system of any one of claims 7 to 10, further comprising means for providing thermal stimuli to a user.

12. The multisensory system of claim 11, wherein the tactile display cells are configured to provide thermo-tactile haptic stimuli to a user.

13. Use of the multisensory system of any one of claims 7 to 12 for Yoga training, meditation or hypnosis' induction.

14. A method for performing Yoga training, meditation, massage or hypnosis' induction, the method comprising the steps of:
a) allowing a user to contact the haptic device of the multisensory system of any one of claims 7 to 12;
b) reproducing an audio file via the audio device of the multisensory system; and
c) operating the haptic device of the multisensory system according to the method of any one of claims 1 to 6.

15. The method of claim 14, wherein the reproduced audio file is the same used for obtaining a haptic profile.

## Patentansprüche

1. Computerimplementiertes Verfahren zum Betreiben einer haptischen Vorrichtung, um das Gefühl bereitzustellen, dass sich eine Welle auf der Haut des Benutzers bewegt, wobei die haptische Vorrichtung eine Mehrzahl von taktilen Anzeigezellen umfasst, die dazu ausgelegt sind, einem Benutzer haptische Reize bereitzustellen, wobei das Verfahren die folgenden Schritte umfasst:
a) Verarbeiten eines Audiosignals, das aus einer Eingabe-Audiodatei von Wasserwellen abgeleitet ist, um dadurch mindestens ein Profil von Frequenzen und Amplituden des Audiosignals zu erhalten;
b) Umwandeln der Frequenz- und Amplitudenprofile in ein haptisches Profil; und
c) Betreiben der haptischen Vorrichtung gemäß dem haptischen Profil, um das Gefühl bereitzustellen, dass sich eine Welle auf der Haut eines Benutzers bewegt,
wobei
- Schritt a) Extrahieren einer Hüllkurve des Audiosignals und Bestimmen der lokalen Maxima und Minima der Hüllkurve umfasst, um dadurch die Anstiegs- und Abfallzeit der Welle abzuleiten.

2. Verfahren nach Anspruch 1, wobei Schritt c) Aktivieren der taktilen Anzeigezellen in einer räumlichzeitlichen Weise umfasst, so dass eine maximale Amplitude der Welle durch einen maximalen taktilen Druckwert normalisiert wird.

3. Verfahren nach Anspruch 1 oder 2, wobei Schritt a) Anwenden eines Bandpassfilters auf die Eingabe-Audiodatei, das grob um eine gewünschte Frequenz zentriert ist, Gleichrichten des Signals und Anwenden eines Tiefpassfilters umfasst.

4. Verfahren nach einem der vorhergehenden Ansprüche, wobei Schritt a) Anwenden einer Fensterfunktion, wie etwa eines gefalteten Hanning-Fensters, umfasst.

5. Verfahren nach einem der vorhergehenden Ansprüche, wobei Schritt c) ferner Bereitstellen thermischer Reize für einen Benutzer über die thermotaktilen Anzeigezellen der haptischen Vorrichtung umfasst.

6. Verfahren nach einem der vorhergehenden Ansprüche, wobei die haptische Vorrichtung 3 Zellen umfasst und für eine gegebene Anstiegszeit der Welle, tr, die Dauer der Stimulation der Zellen, DoS, und die Stimulus-Onset-Asynchronie, SOA, basierend auf dem Prozentsatz der Überlappung, Op, mit den folgenden Gleichungen bestimmt werden: $tr = 3 \times DoS \times OP$ (1) $SOA = \left(1-OP\right) \times DoS .$ (2)

7. Multisensorisches System, umfassend:
a) eine haptische Vorrichtung, die eine Mehrzahl von taktilen Anzeigezellen umfasst, die dazu ausgelegt sind, einem Benutzer haptische Reize bereitzustellen;
b) eine Datenverarbeitungseinrichtung, die mit der haptischen Vorrichtung wirkverbunden ist, wobei die Einrichtung einen Prozessor umfasst, der dazu ausgelegt ist, das Verfahren nach Anspruch 1 bis 6 durchzuführen; und
c) mindestens eine Audiovorrichtung, die dazu ausgelegt ist, eine Audiodatei wiederzugeben.

8. Multisensorisches System nach Anspruch 7, ferner umfassend d) ein computerlesbares Speicherungsmedium, das mindestens eine Audiodatei umfasst.

9. Multisensorisches System nach Anspruch 7 oder 8, wobei die haptischen Reize Kraft und/oder Druck sind.

10. Multisensorisches System nach einem der Ansprüche 7 bis 9, wobei die taktilen Anzeigen pneumatisch oder hydraulisch betrieben werden.

11. Multisensorisches System nach einem der Ansprüche 7 bis 10, ferner umfassend Mittel zum Bereitstellen thermischer Reize für einen Benutzer.

12. Multisensorisches System nach Anspruch 11, wobei die taktilen Anzeigezellen dazu ausgelegt sind, einem Benutzer thermotaktile haptische Reize bereitzustellen.

13. Verwendung des multisensorischen Systems nach einem der Ansprüche 7 bis 12 für Yoga-Training, Meditation oder Hypnose-Induktion.

14. Verfahren zur Durchführung von Yoga-Training, Meditation, Massage oder Hypnose-Induktion, wobei das Verfahren die folgenden Schritte umfasst:
a) Ermöglichen, dass ein Benutzer die haptische Vorrichtung des multisensorischen Systems nach einem der Ansprüche 7 bis 12 berührt;
b) Wiedergeben einer Audiodatei über die Audiovorrichtung des multisensorischen Systems; und
c) Betreiben der haptischen Vorrichtung des multisensorischen Systems gemäß dem Verfahren nach einem der Ansprüche 1 bis 6.

15. Verfahren nach Anspruch 14, wobei die wiedergegebene Audiodatei dieselbe ist, die zum Erhalten eines haptischen Profils verwendet wird.

## Revendications

1. Procédé mis en œuvre par ordinateur pour faire fonctionner un dispositif haptique afin de fournir la sensation de mouvement d'une onde sur la peau de l'utilisateur, le dispositif haptique comprenant une pluralité de cellules d'affichage tactiles configurées pour fournir des stimuli haptiques à un utilisateur, le procédé comprenant les étapes suivantes :
a) traiter un signal audio dérivé d'un fichier audio d'entrée d'ondes d'eau, obtenant ainsi au moins un profil de fréquences et d'amplitudes du signal audio ;
b) convertir des profils de fréquences et d'amplitudes en un profil haptique ; et
c) faire fonctionner le dispositif haptique en fonction du profil haptique pour procurer la sensation de mouvement d'une vague sur la peau d'un utilisateur
où
- l'étape a) comprend d'extraire une enveloppe du signal audio ; et de déterminer des maxima et minima locaux de l'enveloppe, en dérivant ainsi les temps de montée et de descente de l'onde.

2. Procédé selon la revendication 1, dans lequel l'étape c) comprend d'activer des cellules d'affichage tactiles d'une manière spatio-temporelle de sorte qu'une amplitude maximale de l'onde soit normalisée par une valeur de pression tactile maximale.

3. Procédé selon la revendication 1 ou la revendication 2, dans lequel l'étape a) comprend d'appliquer un filtre passe-bande sur le fichier audio d'entrée, largement centré autour d'une fréquence souhaitée ; de rectifier ledit signal ; et d'appliquer un filtre passe-bas.

4. Procédé selon l'une quelconque des revendications précédentes, dans lequel l'étape a) comprend d'appliquer une fonction de fenêtre telle qu'une fenêtre de Hanning convoluée.

5. Procédé selon l'une quelconque des revendications précédentes, dans lequel l'étape c) comprend en outre de fournir des stimuli thermiques à un utilisateur par l'intermédiaire des cellules d'affichage thermotactiles du dispositif haptique.

6. Procédé selon l'une quelconque des revendications précédentes, dans lequel le dispositif haptique comprend 3 cellules et, pour un temps de montée donné de l'onde tr, la durée de stimulation des cellules, DoS, et l'asynchronie d'apparition du stimulus, SOA, sont déterminées en fonction du pourcentage de chevauchement, Op, selon les équations suivantes : $tr = 3 \times DoS \times Op$ (1) $SOA = \left(1-Op\right) \times DoS .$ (2)

7. Système multisensoriel comprenant :
a) un dispositif haptique comprenant une pluralité de cellules d'affichage tactiles configurées pour fournir des stimuli haptiques à un utilisateur ;
b) un appareil de traitement de données connecté fonctionnellement au dispositif haptique, l'appareil comprenant un processeur configuré pour mettre en œuvre le procédé selon les revendications 1 à 6 ; et
c) au moins un dispositif audio configuré pour reproduire un fichier audio.

8. Système multisensoriel selon la revendication 7, comprenant en outre d) un support de stockage lisible par ordinateur comprenant au moins un fichier audio.

9. Système multisensoriel selon la revendication 7 ou la revendication 8, dans lequel les stimuli haptiques sont une force et/ou une pression.

10. Système multisensoriel selon l'une quelconque des revendications 7 à 9, dans lequel les affichages tactiles sont actionnés pneumatiquement ou hydrauliquement.

11. Système multisensoriel selon l'une quelconque des revendications 7 à 10, comprenant en outre des moyens pour fournir des stimuli thermiques à un utilisateur.

12. Système multisensoriel selon la revendication 11, dans lequel les cellules d'affichage tactiles sont configurées pour fournir des stimuli haptiques thermotactiles à un utilisateur.

13. Utilisation du système multisensoriel selon l'une quelconque des revendications 7 à 12 destiné à l'entraînement au yoga, à la méditation ou à l'induction d'hypnose.

14. Procédé pour effectuer de l'entrainement de yoga, de la méditation, un massage ou de l'induction d'hypnose, le procédé comprenant les étapes suivantes :
a) permettre à un utilisateur d'entrer en contact avec le dispositif haptique du système multisensoriel de l'une quelconque des revendications 7 à 12 ;
b) reproduire un fichier audio par l'intermédiaire du dispositif audio du système multisensoriel ; et
c) faire fonctionner le dispositif haptique du système multisensoriel conformément au procédé de l'une quelconque des revendications 1 à 6.

15. Procédé selon la revendication 14, dans lequel le fichier audio reproduit est le même que celui utilisé pour obtenir un profil haptique.
